# FASCICULE DE BREVET EUROPEEN

(11) **EP 2 496 211 B1**
(45) Date de publication et mention de la délivrance du brevet: **21.09.2016**
(21) Numéro de dépôt: 10801180.0
(22) Date de dépôt: 02.11.2010
(51) Int. Cl.: A61K 8/64

(54) **UTILISATION D'UNE COMPOSITION COMPRENANT UN HYDROLYSAT PEPTIDIQUE DE RIZ NON FERMENTE POUR STIMULER LA POUSSE DES CHEVEUX**
VERWENDUNG EINER ZUSAMMENSETZUNG BEINHALTEND EIN NICHT FERMENTIERTES REISPEPTIDHYDROLYSAT ZUR STIMULIERUNG VON HAARWUCHS
USE OF A COMPOSITION COMPRISING A NON FERMENTED RICE PEPTIDE HYDROLYSATE FOR THE STIMULATION OF HAIR GROWTH

(30) Priorité: 03.11.2009 FR 0905256
(43) Date de publication de la demande: 12.09.2012
(73) Titulaire: ISP Investments Inc., Wilmington, DE 19805 (US)
(72) Inventeur: DAL FARRA, Claude, Kerhonkson NY 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2010/000722
(87) Numéro de publication internationale: WO 2011/055032

(56) Documents cités:
- EP-A1- 1 656 970
- WO-A1-2010/055833
- WO-A2-2011/015796
- DE-A1-102004 001 267
- FR-A1- 2 684 295
- FR-A1- 2 888 725
- FR-A1- 2 895 261
- FR-A1- 2 915 380
- FR-A1- 2 915 381
- FR-A1- 2 915 383
- FR-A1- 2 915 384
- FR-A1- 2 944 796
- JP-A- 5 306 211
- JP-A- 2004 099 503
- Kelisema: "Kelisema S.r.l. - Active Ingredients for the Cosmetic Industry", , 31 août 2005 (2005-08-31), pages 1-48, XP002581578, Extrait de l'Internet: URL:www.prochem.ch/html/Kelisema.ppt [extrait le 2010-05-07]
- TSURUKI T ET AL: "Anti-alopecia mechanisms of soymetide-4, an immunostimulating peptide derived from soy beta-conglycinin", PEPTIDES, ELSEVIER, AMSTERDAM, vol. 26, no. 5, 1 mai 2005 (2005-05-01), pages 707-711, XP004820277, ISSN: 0196-9781, DOI: DOI:10.1016/J.PEPTIDES.2005.01.010

## Description

La présente invention se situe dans le domaine des compositions cosmétiques appliquées aux cheveux. La présente invention concerne l'utilisation cosmétique d'une composition comprenant au moins un hydrolysat peptidique de riz non fermenté provenant de l'hydrolyse des protéines des grains de riz débarrassés de leur enveloppe par une étape de décorticage, qui comprend au moins 70 % de peptides de taille inférieure à 6 kDa, en tant qu'agent actif pour freiner, limiter la chute des cheveux et/ ou stimuler leur croissance en augmentant la quantité de protéines marqueurs des cellules souches adultes folliculaires et en favorisant la différenciation de ces cellules.

Les cheveux sont des annexes kératiniques au même titre que les poils, les cils, les sourcils ou encore les ongles. Ils ont un rôle physiologique de protection du cuir chevelu, mais surtout un rôle social, et ce, depuis longtemps dans l'histoire de l'homme. Les cheveux peuvent être de différentes natures, longs, courts, raides, bouclés....mais ils obéissent tous à la loi du cycle. En effet, les 100 000 à 150 000 follicules pileux qui forment une chevelure « normale » se renouvellent tous de façon cyclique, asynchrone et stochastique à partir d'un réservoir de cellules souches adultes folliculaires.

Le follicule pileux est une annexe cutanée autonome avec son propre contrôle hormonal, son propre cycle, une structure complexe et stable (Bernard B. A. ; Médecine/Sciences 2006 ; 22 : 138-43). Le cycle du cheveu se décortique en 3 phases : les phases anagène, catagène et télogène. La phase anagène, ou phase de croissance du cheveu, dure entre 3 et 7 ans (variable selon l'âge, le sexe et la zone du cuir chevelu). Cette phase est suivie par une phase de repos appelée catagène et dure environ 3 semaines. Alors que des processus d'apoptose ont lieu durant cette phase catagène, entraînant ainsi la chute du cheveu, la phase suivante appelée télogène va permettre à un nouveau bulbe de se former à partir d'un germe pileux qui va initier le cycle suivant (Arouete J., J. Med. Esth. Et Chir. Derm., Sept. 2005, 119, 165-167). Cette phase télogène durera, quant à elle, environ 3 mois. C'est ainsi que, sur une chevelure « normale », environ 85 % des follicules sont en phase de croissance, 2 % en phase de repos, et un peu plus de 10 % en phase de chute.

Aussi bien chez les hommes que chez les femmes, on constate qu'il est normal de perdre de 100 à 150 cheveux par jour. Les cheveux tombent et se renouvellent d'eux mêmes. Mais lorsque la chute dépasse largement les 150 cheveux, ou lorsque le renouvellement par un autre follicule est défaillant (cheveux très fins ou raréfiés), on parle alors de chute de cheveux, encore appelée alopécie. Par alopécie, on entend la chute des cheveux ou des poils de manière totale ou partielle, définitive ou transitoire, due notamment à l'âge, à des facteurs génétiques ou faisant suite à une affection locale ou générale... On distingue différents types d'alopécie en fonction de l'origine du trouble :
- l'alopécie androgénétique (souvent héréditaire) est la plus fréquente : elle se manifeste par une diminution du volume des cheveux, voire une calvitie, et touche 70% des hommes (mais elle atteint également les femmes) ;
- l'alopécie aiguë : elle peut être liée à un traitement par chimiothérapie, un stress, des carences alimentaires importantes, une carence en fer, des troubles hormonaux, une irradiation aiguë ;
- l'alopécie localisée : elle peut être provoquée par des problèmes de peau (tumeur, brûlure, pelade), une radiothérapie ou des parasites (teigne, lichen) ;
- l'alopécie congénitale ;
- l'alopécie Areata qui semble être d'origine auto-immune (mécanisme de médiation cellulaire) qui se caractérise par une atteinte en "patch" plus ou moins gros et à un ou plusieurs endroits. Cette forme de pelade peut atteindre toute la tête, on parle d'alopécie Totalis, et parfois l'ensemble du corps, on parle alors d'alopécie Universalis (dans ce cas, il n'y a plus aucun poils ni cheveux sur l'ensemble du corps).

Différents traitements cosmétiques et/ou médicamenteux ont été développés ces dernières années afin de traiter au mieux les différentes alopécies. L'alopécie androgénétique est celle pour laquelle le plus de traitements existent, étant donné que c'est l'alopécie qui touche le plus de monde. L'origine de ce type d'alopécie est due à une trop grande sensibilité aux hormones masculines ou androgènes, elle-même due à l'hérédité. Sous l'influence d'une enzyme, la 5-alpha-réductase, la testostérone se transforme en dihydrotestostérone ou DHT qui va stimuler les glandes sébacées. Il se créera ainsi une séborrhée permanente qui va entraîner l'obstruction progressive du follicule pileux et l'asphyxie du bulbe, ayant pour conséquence l'arrêt brutal de la phase anagène.

Parmi les traitements proposés pour le traitement de ce type d'alopécie, on peut citer le Minoxidil, ou encore le Propecia®. Le premier permet de retarder la fin des cycles de croissance et d'agir sur la perte des cheveux de manière indirecte. Cependant, il compte de nombreux inconvénients comme le fait qu'il doive être administré très tôt lors de l'apparition de l'alopécie, ou encore que, dès l'arrêt du traitement la chute recommence. Le Propecia®, ou finastéride, est un agent bloqueur de l'enzyme responsable de la conversion de la testostérone en dihydrotestostérone au niveau des follicules pileux. Il permet de réduire la chute des cheveux et d'activer leur repousse. Mais l'un des inconvénients majeurs de ce composé réside dans son risque à provoquer une augmentation de l'incidence des cancers de la prostate. De plus, sa contre-indication pour le traitement de l'alopécie chez les femmes réduit son champ d'application.

Des compositions orientées cosmétiques ont été proposées afin de pallier au manque de traitements médicaux de l'alopécie androgénétique. Certaines compositions sont plus ou moins destinées à augmenter le volume ou la densité apparente des cheveux. D'autres compositions sont destinées à plus ou moins stimuler la pousse et la santé des cheveux par augmentation de la synthèse des protéines clés dans la formation du cheveu. Cependant, il existe à l'heure actuelle une forte demande quant à des compositions permettant de limiter la chute des cheveux et/ou stimuler leur pousse.

C'est ainsi que la Demanderesse a mis en évidence les propriétés en tant qu'agent antichute d'un extrait particulier de riz, particulier en ce que l'extrait est un hydrolysat peptidique provenant de l'hydrolyse des protéines des grains de riz, ledit riz étant non fermenté.

Le riz est connu depuis longtemps en tant qu'agent stimulant la pousse des cheveux avec plus ou moins d'efficacité. Par exemple, une composition, comprenant un extrait de riz en tant que promoteur de la production du facteur de croissance HGF, a été décrite comme activateur de la pousse des cheveux (brevet JP 2004099503A). Un brevet japonais déposé par SO-KEN KK (JP5306211A) décrit une composition tonique ayant des propriétés antichute des cheveux, ladite composition comprenant un extrait aqueux ou organique de riz obtenu par décomposition par une amylase. D'autres publications et/ou demandes de brevet décrivent des extraits de riz stimulant la pousse des cheveux, comme la demande de brevet FR 2684295. Dans cette demande, l'extrait de riz est obtenu à partir du son du grain de riz et suivi par une étape de fermentation. Cependant, aucun de ces extraits n'est un extrait peptidique issu de l'hydrolyse des protéines des grains de riz non fermentés pour lutter contre la chute des cheveux et préserver leur santé. L'art antérieur le plus proche de l'invention consiste en un extrait peptidique de riz, destiné au traitement et à la prévention du vieillissement de la peau (demandes de brevet FR 2915379, FR 2915380, FR 2915383). Dans ces documents il n'est jamais décrit ou suggéré que les compositions divulguées sont destinées au traitement de la chute des cheveux.

Ainsi, la présente invention se rapporte à l'utilisation cosmétique d'une composition comprenant au moins un hydrolysat peptidique de riz non fermenté provenant de l'hydrolyse des protéines des grains de riz débarrassés de leur enveloppe par une étape de décorticage, qui comprend au moins 70 % de peptides de taille inférieure à 6 kDa, en tant qu'agent actif pour freiner, limiter la chute des cheveux et/ou stimuler leur croissance en augmentant la quantité de protéines marqueurs des cellules souches adultes folliculaires et en favorisant la différenciation de ces cellules.

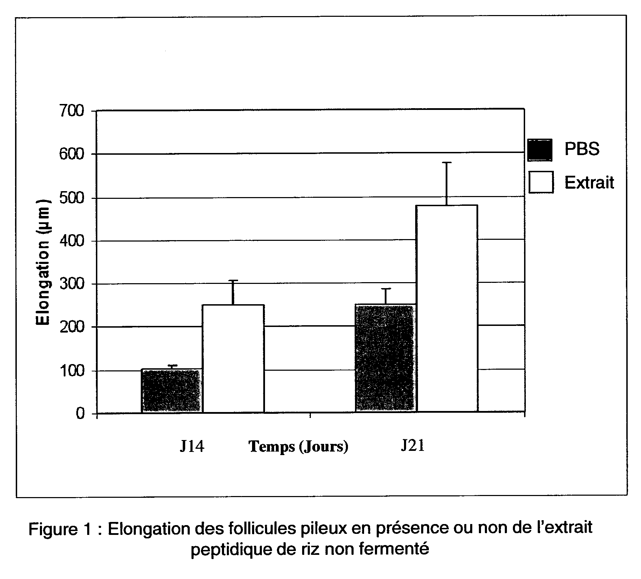

La figure 1 représente un histogramme compilant et comparant les résultats d'une étude d'élongation des follicules pileux en présence ou non de l'hydrolysat peptidique de riz non fermenté selon l'invention.

Selon l'invention, on utilisera indifféremment les termes « hydrolysat peptidique », « extrait », « extrait peptidique », « extrait solubilisé » ou « agent actif ».

On entend par « hydrolysat peptidique », un extrait obtenu par hydrolyse des protéines des grains de riz, comprenant un mélange de composés majoritairement représentés par des peptides ou des oligopeptides. Par « hydrolysat peptidique de riz non fermenté», on entend un hydrolysat obtenu à partir des protéines des grains de riz décortiqués n'ayant pas subi d'étape de fermentation, ni avant les hydrolyses, ni après l'obtention de l'hydrolysat.

La Demanderesse a découvert qu'un extrait peptidique de riz non fermenté présente une activité certaine sur les follicules pileux des cheveux et, en particulier, avait une action en tant qu'agent antichute. De nombreux extraits de riz existent dans cette indication mais ces extraits sont la plupart du temps des extraits glucosidiques, les protéines et peptides présents étant délibérément détruits lors de l'obtention de l'extrait. L'utilisation d'un extrait non fermenté a permis à la Demanderesse d'optimiser les performances biologiques de celui-ci, en termes quantitatif et qualitatif, par rapport aux extraits de riz existants. Grâce à la non-fermentation des grains de riz, les protéines les composant ne sont pas dégradées par les protéases des levures de fermentation. Ainsi, on conserve un maximum de protéines natives et il sera aisé de les hydrolyser de manière sélective et spécifique afin d'obtenir un grand nombre de peptides bioactifs.

On entend par « agent actif pour freiner, limiter la chute des cheveux et/ou stimuler leur croissance », tout hydrolysat peptidique de riz non fermenté pouvant stimuler le follicule de cheveu, l'expression de α6-intégrine, les marqueurs de différenciation des kératinocytes telles que la kératine K15, ou encore la β-caténine, ou encore les marqueurs de prolifération comme p63.

Les fibres kératiniques humaines auxquelles s'applique l'invention sont notamment les cheveux, les sourcils, les cils, les poils de barbe, de moustache, les poils pubiens et les ongles. Selon un mode particulier de réalisation, la composition est destinée à être utilisée pour activer la pousse des cils.

L' utilisation d'au moins un hydrolysat peptidique de riz selon l'invention, en tant qu'agent actif pour protéger les cellules souches adultes folliculaires ainsi que leur micro- environnement spécifique est divulguée. L'hydrolysat a été testé par la Demanderesse, et celle-ci a pu démontrer l'action biologique de celui-ci sur les cellules formant le follicule pileux, notamment les cellules souches adultes folliculaires. Il a été montré que l'extrait avait plus particulièrement une action sur le micro-environnement spécifique constituant la « niche » qui protège lesdites cellules souches. Enfin, il a été démontré une action de protection de ces mêmes cellules par ledit extrait.

Il est connu que les cellules souches adultes, notamment folliculaires, sont difficiles à identifier. Cependant, elles sont néanmoins distinguables car elles expriment un ensemble de marqueurs qui permet d'obtenir un profil spécifique d'expression pour ce type cellulaire. Parmi ces marqueurs, on retrouve entre autre :
- la kératine K15 qui est un marqueur de cellules souches au stade le plus différencié dans le compartiment folliculaire appelé bulge (Bernard B. A. ; Médecine/Sciences 2006 ; 22 : 138-43);
- l'α6 intégrine (qui colocalise avec la Laminine-5) qui est décrite comme étant un marqueur potentiel de cellules souches folliculaires (Bernard B. A. ; Médecine/Sciences 2006 ; 22 : 138-43);
- la β-caténine qui joue un rôle clé dans la différenciation et la pousse du follicule du cheveu (DasGupta and Fuchs, 1999, Development 126 :4557-68; Van Mater et al., 2003, Gene dev.17 :1219-24) ;
- p63 qui est un marqueur lié à la capacité proliférative des cellules souches à générer un follicule, une glande sébacée et un épiderme (Chikh et al., 2007, Biochem Biophys Res Commun.,14;361(1):1-6.

L'hydrolysat a démontré son action sur les différents marqueurs cités ci-dessus. En effet, on assiste à une hausse de l'expression de ces marqueurs lorsqu'une composition comprenant au moins ledit hydrolysat est appliquée sur des follicules en culture. La conséquence de ces surexpressions se traduit en une limitation de la chute des cheveux, et par la suite, en une stimulation de la pousse de ceux-ci.

L' utilisation d'au moins un hydrolysat selon l'invention en tant qu'agent actif capable de lutter contre le vieillissement et, en particulier, le photo-vieillissement des cheveux est divulguée. Il est connu que le vieillissement des cheveux se manifeste essentiellement (outre leur blanchiment) par une baisse de la densité capillaire et par la diminution progressive du diamètre des follicules, donnant à la chevelure un aspect plus pauvre, plus clairsemé (Pelfini, C. et al., J. Méd. Esth. Et Chir. Derm.1987 ; Birch MP et al. Br. J. Dermatol 2001;144:297-304). De plus, outre le vieillissement classique, génétiquement programmé, il y a le photo-vieillissement qui lui est causé par l'accumulation d'agressions dues aux rayonnements UV et qui aboutissent à une détérioration prématurée de la structure des cheveux et à un épuisement des follicules. Par conséquent, l'utilisation d'un extrait qui permet de protéger les cellules souches adultes folliculaires aura pour effet de prévenir le vieillissement, en particulier le vieillissement prématuré, et notamment le photo-vieillissement.

Selon un mode avantageux de réalisation de l'invention, l'hydrolysat provient de l'hydrolyse des protéines des grains du riz *Oryza Sativa L.* L'hydrolysat peptidique est constitué par un mélange de composés majoritairement représentés par des peptides. Le terme « peptide » désigne un enchaînement de deux ou plusieurs acides aminés liés entre eux par des liaisons peptidiques ou par des liaisons peptidiques modifiées ; le terme « polypeptide » désignant un peptide de taille plus importante. L'utilisation d'hydrolysats peptidiques, et en particulier d'hydrolysats peptidiques de bas poids moléculaires, présente de nombreux avantages en cosmétique. Outre le fait de générer des composés de nature peptidique qui ne préexistaient pas dans le mélange protéique de départ, l'hydrolyse et la purification permettent d'obtenir des mélanges plus stables, plus facilement standardisables et ne provoquant pas de réactions allergiques en dermato-cosmétique.

L'hydrolysat selon l'invention est obtenu par extraction des protéines du riz non fermenté, extraction suivie d'une hydrolyse contrôlée qui libère les fragments peptidiques biologiquement actifs. De très nombreuses protéines trouvées dans les plantes sont susceptibles de contenir des fragments peptidiques biologiquement actifs au sein de leur structure. L'hydrolyse ménagée permet de dégager ces fragments peptidiques. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les fragments peptidiques ensuite. Il est également possible d'utiliser certains extraits hydrolysés sans en purifier les fragments peptidiques correspondant aux peptides biologiquement actifs selon l'invention, mais en s'assurant toutefois de la présence desdits fragments par des moyens analytiques appropriés.

### Protocole d'extraction

Pour réaliser l'extraction, on utilise les grains de riz décortiqués. Selon l'invention, on utilise une des nombreuses plantes de la famille des poacées du genre Oryza (riz), et préférentiellement l'espèce *Oryza sativa L.* Selon l'invention, le matériel végétal utilisé sera le grain débarrassé de son enveloppe par une étape de décorticage.

Dans une première étape, les grains de riz décortiqués sont broyés à l'aide d'un broyeur à plantes. La poudre ainsi obtenue peut ultérieurement être "délipidée" à l'aide d'un solvant organique classique (comme par exemple un alcool, l'hexane ou de l'acétone). Comme dit précédemment, il n'y a aucune étape de fermentation subie par les grains, ainsi l'extrait reste concentré en protéines natives.

On réalise ensuite l'extraction des protéines suivant un procédé classique (Osborne, 1924) modifié ; le broyat de plantes est mis en suspension dans une solution alcaline contenant un produit adsorbant de type polyvinylpolypyrrolidone (PVPP) insoluble (0,01 -20 %) ; en effet, il a été observé que les opérations d'hydrolyses et de purifications ultérieures étaient facilitées par ce moyen. En particulier, la concentration en substances de type phénoliques, interagissant avec les protéines, se trouve nettement réduite.

La fraction soluble, contenant les protéines, les glucides et éventuellement des lipides, est recueillie après des étapes de centrifugation et de filtration. Cette solution brute est ensuite hydrolysée dans des conditions ménagées pour générer des peptides. L'hydrolyse se définit comme étant une réaction chimique impliquant le clivage d'une molécule par de l'eau, cette réaction pouvant se faire en milieu neutre, acide ou basique. Selon l'invention, l'hydrolyse est réalisée par voie chimique et/ou de façon avantageuse par des enzymes protéolytiques. On peut alors citer l'utilisation des endoprotéases d'origine végétale (papaïne, bromelaïne, ficine) ou de micro-organismes (Aspergillus, Rhizopus, Bacillus, alcalase® de Novozym, etc.).

Pour les mêmes raisons que précédemment, c'est-à-dire l'élimination des substances polyphénoliques, une quantité de polyvinylpolypyrrolidone est additionnée au milieu réactionnel lors de cette étape d'hydrolyse ménagée. Après l'étape de filtration qui permet d'éliminer les enzymes et les polymères, le filtrat (solution) obtenu constitue une première forme de l'agent actif selon l'invention.

On procède alors à une phase de dilution. Ainsi, l'extrait est solubilisé dans un ou plusieurs solvants physiologiquement acceptables, comme l'eau, le glycérol, l'éthanol, le propylène glycol, le butylène glycol, le dipropylène glycol, les diglycols éthoxylés ou propoxylés, les polyols cycliques, ou tout mélange de ces solvants. Cette étape de dilution est suivie d'une stérilisation par ultrafiltration afin d'obtenir un extrait peptidique caractérisé par une teneur composés de nature peptidique de 1,5 à 4 g/l. Préférentiellement, l'extrait obtenu comprend une teneur en ces mêmes composés comprise entre 2 et 3 g/l.

L'hydrolysat obtenu à ce stade est encore purifié afin de sélectionner les fractions de bas poids moléculaires, préférentiellement inférieures à 6 kDa. Ainsi, au moins 70 % des composés de nature peptidique présents dans l'extrait sont des peptides de taille inférieure à 6 kDa, et préférentiellement au moins 85%. La purification s'effectue avantageusement par des étapes d'ultrafiltrations successives à travers des filtres de porosité décroissante, en conservant les filtrats à chaque étape et/ ou par une méthode de type chromatographique.

Ainsi, selon un mode de réalisation avantageux de l'invention, l'extrait peptidique a un pH compris entre 4 et 7, et préférentiellement entre 5 et 6, un extrait sec titrant entre 2 et 5 g/l, et de manière préférée entre 3 et 4 g/l, sa teneur en composés de nature peptidique est comprise entre 1,5 et 4g/l, et préférentiellement entre 2 et 3 g/l, et sa teneur en sucres est de 0,5 à 1 g/l.

Selon un second mode de réalisation de l'invention, l'extrait solubilisé peut être encapsulé ou mis dans un vecteur cosmétique ou pharmaceutique comme les liposomes ou toute autre microcapsule utilisée dans le domaine de la cosmétique, ou adsorbé sur des polymères organiques poudreux, des supports minéraux comme les talcs et bentonites, et plus généralement solubilisé dans, ou fixé sur, tout vecteur physiologiquement acceptable.

Ainsi, l'extrait solubilisé est utilisé dans les compositions selon l'invention à une concentration comprise entre 0,001 % à 5 % environ, et préférentiellement à une concentration comprise entre 0,01 % et 1 % environ par rapport au poids total de la composition finale.

Préférentiellement, la composition utilisé selon l'invention se présente sous une forme adaptée à l'application par voie topique.

La composition utilisée selon l'invention peut en particulier consister en une composition pour soins capillaires, et notamment un shampooing, un après-shampooing, une lotion de mise en plis, une lotion traitante pré- ou post- traitement agressif pour les cheveux, une crème ou un gel coiffant, une lotion restructurante pour les cheveux, un masque, de mousse traitante etc.

La composition pourra notamment être sous forme de crème, émulsion huile-dans-eau, eau-dans-huile, ou émulsions multiples de type huile-dans-eau-dans-huile ou eau-dans-huile-dans-eau, suspension, gel aqueux, solutions aqueuses, hydroalcooliques, ou huileuses. La composition peut être plus ou moins fluide et se présenter sous la forme d'une crème blanche ou colorée, d'une pommade, d'un lait, d'une lotion, d'un sérum, d'une mousse, d'une biphase, ou encore sous forme d'un aérosol.

Enfin, la composition pourra comprendre tout additif communément utilisé dans le domaine d'application envisagé ainsi que les adjuvants nécessaires à leur formulation, tels que des co-solvants (éthanol, glycérol, alcool benzylique, humectant...), des épaississants, des diluants, des émulsionnants, des anti-oxydants, des colorants, des filtres solaires, des pigments, des charges, des conservateurs, des parfums, des absorbeurs d'odeur, des huiles essentielles, des oligo-éléments, des acides gras essentiels, des tensioactifs, des polymères filmogènes, des filtres chimiques ou minéraux, des agents hydratants ou des eaux thermales etc. On peut, par exemple, citer des polymères hydrosolubles de type polymère naturel, tels que les polysaccharides, ou polypeptides, des dérivés cellulosiques de type méthylcellulose ou hydroxypropylcellulose, ou encore des polymères synthétiques, polaxamères, carbomères, PVA ou PVP, et notamment les polymères vendus par la société ISP.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition selon l'invention est une émulsion, la phase grasse peut représenter de 5 à 80 % en poids, et de préférence de 5 à 50 %, par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids par rapport au poids total de la composition.

Par ailleurs, la composition utilisée selon l'invention peut comprendre, en outre, au moins un composé améliorant la pousse et/ou la santé des cheveux.

On peut citer notamment des vitamines, d'autres hydrolysats peptidiques végétaux, le minoxidil, des esters de l'acide nicotinique, des oligo-éléments, des agents anti-inflammatoires, de l'acide rétinoïque ou ses dérivés, du rétinol, des inhibiteurs de la 5α-réductase ou des composés peptidiques issus de la synthèse chimique. Comme exemple de vitamines on peut citer les vitamines A, E, B5, B6, C, H, ou PP ; comme exemple d'oligo-éléments on peut citer le zinc, le cuivre, le magnésium, ou encore le silicium.

Un mode de réalisation particulier de l'invention se rapporte à l'utilisation d'une composition comprenant outre l'hydrolysat peptidique de riz non fermenté, un hydrolysat peptidique de soja. Des tests réalisés sur des biopsies de peaux ont montré l'effet protecteur de cette association d'extraits vis-à-vis des rayonnements UV, et ces résultats peuvent être extrapolés et appliqués aux cellules de l'outer root sheet ou ORS.

Au final, la composition utilisée selon l'invention est composée entre 10 et 90 % d'un hydrolysat de riz non fermenté, et entre 10 et 90 % d'un hydrolysat de soja. Préférentiellement, la composition comprend respectivement 90 % d'hydrolysat de riz non fermenté et 10 % d'hydrolysat de soja. Les hydrolysats de riz et soja peuvent être obtenus séparément et ensuite incorporés dans la composition, ou encore être réalisés en même temps en un seul et unique hydrolysat, cet unique hydrolysat étant par la suite incorporé à la composition.

Egalement décrit est un procédé de traitement non thérapeutique destiné à freiner, limiter la chute des cheveux et/ou stimuler leur croissance, caractérisé en ce que l'on applique quotidiennement sur la zone du cuir chevelu à traiter une composition telle que décrite précédemment.

Egalement décrit est un procédé de traitement non thérapeutique destiné à protéger les cellules souches adultes folliculaires, ainsi que leur micro-environnement spécifique, par application quotidienne sur la zone du cuir chevelu à traiter d'une composition telle que décrite précédemment.

Egalement décrit est un procédé de traitement non thérapeutique destiné à prévenir le vieillissement des cheveux, en particulier le photo-vieillissement, par application quotidienne sur la zone du cuir chevelu à traiter de la composition telle que décrite précédemment.

Comme dit précédemment, le vieillissement des cheveux se manifeste, avant leur disparition parfois totale, par la diminution de la densité capillaire, la miniaturisation des follicules capillaires, la raréfaction et l'amincissement des cheveux et, finalement, le grisonnement aboutissant à la canitie. Or, la composition comprenant l'hydrolysat tel que décrit précédemment a montré qu'elle pouvait limiter le phénomène naturel de vieillissement des cheveux grâce à la préservation des cellules souches notamment, et de leur micro-environnement spécifique. En outre, l'utilisation de la composition en prévention des dommages causés par les rayonnements UV a prouvé son intérêt dans la prévention du photo-vieillissement. C'est ainsi que, par exemple, l'hydrolysat pourrait être avantageusement utilisé dans une composition en tant qu'agent photo-protecteur et, plus particulièrement, en tant qu'agent photo-protecteur dit « secondaire ». En effet, on distingue les agents photo-protecteurs primaires des agents photo-protecteurs secondaires. Les agents photo-protecteurs primaires sont des substances qui exercent un pouvoir physique : ils sont en mesure d'absorber les rayonnements UV et de les restituer sous forme de chaleur afin de protéger la peau et les phanères. Les agents photo-protecteurs secondaires sont des substances qui ont généralement un effet biologique ; ce sont, par exemple, des agents capables de limiter les dommages causés à l'ADN et aux membranes des cellules par la pénétration des rayonnements UV.

Egalement décrit est un procédé de traitement non thérapeutique destiné à prévenir les dommages et à protéger les cheveux et la tige capillaire des agressions extérieures, grâce à l'application d'une composition selon l'invention, et ce avant l'exposition au soleil, à des produits chimiques ou des sources de chaleur, telles que des colorations, des défrisages, des permanentes, ou encore des brushings. On entend, par « agressions extérieures », les agressions que peuvent produire l'environnement. A titre d'exemple, on peut citer des agressions telles que la pollution, les UV, ou encore les produits à caractère irritant tels que les produits de coloration, décoloration, défrisage, les tensioactifs, les conservateurs ou les parfums. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldehyde), ou bien encore la fumée de cigarette. Il est connu, notamment par les professionnels de la coiffure que, par exemple, les traitements chimiques agressent la tige capillaire et que, par conséquent, l'utilisation d'un soin capillaire en pré-traitement permet de protéger les cheveux et le cuir chevelu. A cet effet, l'utilisation d'une composition comprenant un extrait peptidique de riz non fermenté permet donc d'avoir une action préventive et de protection.

D'autres avantages et caractéristiques de l'invention apparaîtront mieux à la lecture des exemples donnés à titre illustratif et non limitatif.

### Exemple 1 : Procédé de préparation d'un extrait peptidique de riz non fermenté

L'extrait utilisé selon l'invention est obtenu à partir de plantes de l'espèce *Oryza sativa L*. Bien entendu, l'extrait peut être préparé à partir de plantes d'au moins l'une quelconque des nombreuses variétés et espèces appartenant au genre *Oryza.*

Dans une première étape, 1 kg de grains de riz décortiqués sont broyés dans un broyeur à céréales. La farine obtenue est délipidée par l'action d'un solvant organique, l'hexane. Après filtration et séchage sous vide, la poudre obtenue est mise en suspension dans une solution aqueuse alcaline (dilution au 1/10) pH 10 contenant 1 % de polyvinylpolypyrrolidone (PVPP, Polyclar V ISP). Ce mélange est maintenu sous agitation pendant un temps suffisamment long pour permettre la solubilisation des fractions solubles. La température d'extraction est variable (comprise entre 4 et 80°C); mais préférentiellement l'opération sera réalisée à froid. Après cette phase d'extraction, le milieu est clarifié par centrifugation puis filtré sur filtre à plaque. Ce filtrat qui contient les fractions solubles du riz est ensuite soumis à une précipitation des protéines en faisant varier la force ionique en milieu neutre ou acide, ce qui permet d'éliminer les composants glucidiques solubles, les lipides et les acides nucléiques. Le pH du milieu est ensuite amené à 3,5. Le surnageant est éliminé et le précipité est ensuite lavé à l'aide d'un solvant tel que, par exemple, l'éthanol ou le méthanol. Enfin, le solvant est évaporé par séchage sous vide.

A ce stade, on obtient environ 50 grammes de poudre de couleur jaune clair d'extrait protéique brut contenant :
- Protéines : 75 %
- Glucides : 20 %
- Lipides : 5 %

Le précipité riche en protéines est remis en solution dans l'eau ou un autre solvant.

L'extrait protéique brut est alors soumis à une série d'hydrolyses ménagées et sélectives consistant en des hydrolyses chimiques et enzymatiques en présence de 0,5 % de PVPP (Polyclar V) et d'endopeptidases à cystéine (papaïne, ficine). Après réaction, l'hydrolysat est filtré sur plaque puis sur cartouche stérilisante (0,2 µm).

On obtient alors un hydrolysat de couleur claire, titrant de 15 à 30 g/l d'extrait sec, qui est alors dilué de telle sorte que la concentration en composés de nature peptidique, déterminée par la méthode de Lowry, soit comprise entre 0,1 et 5g/l, et préférentiellement entre 0,5 et 2 g/l.

On procède ensuite à une ultrafiltration de la solution sur une cartouche de filtration Millipore Helicon (seuil de coupure ; 6 kDa). Les hauts poids moléculaires contenus dans le retentât sont écartés, le filtrat est conservé. Après analyse, notamment par HPLC, on constate que l'extrait contient environ 80 % de composés peptidiques de taille inférieure à 6 kDa.

Une autre variante de procédé d'obtention de l'extrait consiste à effectuer une purification de l'extrait obtenu précédemment, par chromatographie d'échange d'ions, sur une colonne TSK gel (TosoHaas) avec un tampon phosphate pH 7.

L'extrait obtenu selon le procédé décrit dans l'exemple 1 est ensuite solubilisé dans du glycérol.

### Exemple 2 : Procédé de préparation d'un mélange d'un extrait peptidique de riz non fermenté et d'un extrait peptidique de soja

La farine de riz et le tourteau de soja sont préalablement mis en solution, dans des proportions respectives de 90:10, dans 20 volumes d'eau ajustés à un pH compris entre 8.0 et 8.5. Après ajustement du pH, il est rajouté dans le milieu réactionnel 2 % de bromélaïne, 2 % d'alcalase® et 2 % de POLYCLAR® 10 (polyvinylpyrrolidone - PVPP - insoluble). Le milieu réactionnel est ensuite chauffé deux heures à 50°C puis désactivé deux heures à 80°C. Une étape de filtration permet de récupérer le filtrat composé de 20 à 25 g/L de matière sèche, de 18 à 22 g/L de protéines et de 2 à 3 g/L de sucres.

La nature protéique de ce filtrat est mise en évidence par électrophorèse sur gel de polyacrylamide. Pour cette analyse, il est utilisé les gels NuPAGE® Bis-Tris Pre-cast (Invitrogen). L'extrait protéique intermédiaire de riz-soja est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites se réoxydent durant l'électrophorèse. La migration des protéines est réalisée à l'aide du tampon de migration NuPAGE® MES avec le standard SeeBlue Plus2 comme marqueur de poids moléculaire. La coloration des protéines est effectuée à l'aide de Bleu de Coomassie® R-250. Le profil protéique ainsi obtenu met en évidence une répartition des poids moléculaires compris entre 15 et 3 kDa.

L'extrait protéique intermédiaire de riz-soja précédemment obtenu est alors purifié par des filtrations successives à l'aide de filtres-plaques Seitz-Orion de porosité décroissante (jusqu'à 0.2 µm) afin d'obtenir une solution jaune pâle brillante et limpide. A cette étape, l'extrait de riz-soja est caractérisé par un sec de 20-24 g/kg, un taux de protéines de 18-21 g/L et un taux de sucres de 1-3 g/L.

Cette solution est alors purifiée en éliminant les protéines de poids moléculaire supérieur à 5 kDa à l'aide de filtrations à flux tangentiel.

Pour cela, la solution de riz-soja est pompée sous pression à travers un support Pellicon® équipée de cassette Pellicon® 2 Biomax 10 kDa. Ce 1er filtrat est récupéré pour être ensuite filtré à travers une autre cassette Pellicon® 2 Biomax 3 kDa. En fin de purification, on obtient un extrait végétal de riz-soja jaune pâle, brillant et limpide. Il est caractérisé par un sec de 17-20 g/kg, une teneur en protéines de 15-18 g/L et un taux de sucres entre 1 et 2 g/L.

Cette solution est alors analysée en chromatographie liquide haute pression à l'aide d'un appareil HP1100 piloté par le logiciel ChemStation. La colonne utilisée lors de l'élution de l'extrait de riz-soja est une Nucleosil® 300-5 C4 MPN (125 x 4 mn). Cette colonne permet de chromatographier des protéines ayant des poids moléculaires de 0.2 à 25 kDa (selon un gradient de solvants approprié). Dans ces conditions chromatographiques, il a été isolé plusieurs fractions peptidiques. Ces diverses fractions ont été analysées par spectrométrie de masse afin d'identifier leurs pics moléculaires. La détermination de la composition en acides aminés a également été réalisée. Celle-ci est obtenue après hydrolyse acide et identification par chromatographie liquide haute pression à l'aide d'une pré-dérivation au PICT (phenylisothiocyanate).

### Exemple 3 : Action de l'extrait peptidique de riz non fermenté sur des follicules maintenus en culture in vitro

### 3.1 Marquage de la kératine K15

### Cultures de follicules pileux et inclusion des coupes

Des biopsies de peaux (issues de liftings) présentant des cheveux sont cultivées de la même manière que des explants de peaux. Des biopsies de 6 mm sont réalisées au moyen de punch de biopsies et mises en culture sur des inserts dans un milieu WILLIAM E. en présence d'antibiotiques (Pénicilline 100 UI/ml-10 µg, streptomycine, 10 µg/ml d'insuline 10 nG/ml, d'Hydrocortisone et 2 mmol/L de L-Glutamine).

Les explants de peaux sont déposés dans des plaques 6 puits et les follicules sont traités ou non en mettant 20 µl d'extrait à 1 % en contact sur les biopsies, après 48 heures de culture. A la fin de l'expérience, les biopsies sont mises en cassette et plongées dans un mélange de formol à 10 % pendant 2 heures dans un appareil automatisé (VIP). L'enrobage des cellules à l'aide de paraffine est préparé par une série de bains d'alcool (à concentration et temps croissants), suivie de 2 bains de xylène et enfin d'un bain de paraffine. La durée totale de cette série d'opérations est d'une douzaine d'heures. Les biopsies ainsi enrobées sont ensuite placées dans des cassettes appropriées, orientées et mises dans un bloc de paraffine afin d'être ensuite coupées à 4 µm par un microtome. Les coupes paraffinées sont ensuite déparaffinées et réhydratées avant la mise de l'anticorps. Pour cela, une série de bains de xylène, suivie d'une série de bains d'alcool (à concentration et temps croissants) et d'un rinçage dans de l'eau, puis dans du PBS sont réalisés à cet effet.

### Immunomarquage de la Kératine K15

Les coupes déparaffinées sont rincées au PBS pendant 2 minutes, puis entourées, et on incube chaque coupe dans 100 µl de BSA 5 % pendant 30 minutes. Puis, 100 µl d'anticorps primaire Cytokératine K15 (Abcam, Mouse monoclonal) sont ajoutés et mis pendant 60 minutes sous agitation en chambre humide. Après rinçage avec du PBS pendant 30 minutes, 100 µL d'un anticorps secondaire marqué à la fluorescence sont ajoutés et laissés durant 1 heure à l'obscurité sous agitation en chambre humide. Ces marquages sont réalisés en parallèle sur des biopsies traitées ou non avec l'extrait peptidique actif. Les lames sont ensuite rincées dans le PBS, montées entre lame et lamelle dans l'Aquatex, et l'observation s'effectue au microscope à épifluorescence.

### Résultats

Grâce à un logiciel de quantification de la fluorescence, on constate une augmentation de l'ordre de 95,3 % de la kératine K15 dans les follicules pileux traités à l'aide de l'extrait actif par rapport aux follicules non traités. Or, la kératine K15 est impliquée dans les stades précoces de la différentiation des kératinocytes dans les follicules pileux.

### 3.2 Marquage de l'α6 intégrine

Afin de réaliser un marquage de l'α6 intégrine dans les follicules pileux, des biopsies de lifting sont réalisées comme décrits dans l'exemple 3.1. Les explants de peaux sont déposés dans des plaques 6 puits et les follicules sont traités ou non en mettant 20 µl d'extrait actif en contact sur les biopsies, après 48 heures de culture. Par la suite, afin de préparer le marquage par anticorps, les coupes ne sont pas paraffinées mais mises mis dans l'OCT (optimum cutting température) et coupées au cryostat (frozen section). Les biopsies sont donc prises et incluent dans l'OCT qui se solidifie au froid (à -20°C), et les blocs ainsi solidifiés sont ensuite coupés au cryotome où des coupes de 6 µm sont réalisées. Les coupes sont recueillies sur des lames d'observation poly-lysinées. Les coupes avec l'OCT sont mises à température ambiante avant d'être utilisées pour l'immunomarquage.

### Immunomarquage de l'α6 intégrine

Les lames montées dans l'OCT sont séchées à l'étuve à 37°C pendant 30 minutes, puis les coupes sont fixées dans un bain d'acétone (conservé au préalable à -20°C) pendant 10 minutes. Les lames sont rincées dans du PBS pendant 5 minutes, puis les coupes sont entourées par le stylo Pappen. Les coupes sont saturées par ajout de BSA à 5 % pendant 30 minutes, puis le marquage avec l'anticorps primaire est réalisé comme décrit précédemment. Le marquage est réalisé grâce à un anticorps anti-α6 intégrine (Tebu Santa Cruz, mouse monoclonal). Ce premier marquage est suivi par un marquage secondaire par un anticorps marqué à la fluorescence. Le montage de la lame dans un milieu adéquate puis la lecture par un microscope à épifluorescence permettront d'apprécier l'intensité du marquage. Ce marquage est réalisé en parallèle sur des biopsies traitées ou non avec l'extrait actif afin d'apprécier l'effet de ce dernier sur lesdites biopsies.

### Résultats

Le traitement des follicules par l'extrait peptidique issu du riz non fermenté permet d'augmenter fortement la quantité d'α6 intégrine. On constate une hausse de 108 % de la quantité d'α6 intégrine par rapport aux follicules non traités. Or, il est connu que l'α6 intégrine est un des marqueurs potentiels des cellules souches folliculaires.

### 3.3 Marquage de la β-caténine

Le même protocole (que celui décrit dans l'exemple 3.2) a été utilisé afin d'effectuer un marquage de la β-caténine (anticorps primaire anti-béta-caténine Abcam suivi d'un anticorps secondaire marqué à la fluorescence) et, là aussi, on constate une augmentation du marquage de la β-caténine dans les follicules traités avec l'extrait peptidique par rapport aux follicules non traités. Or, il est connu que la β-caténine joue un rôle clé dans la différenciation et la croissance du follicule pileux.

### 3.4 Marquage de la protéine p63

Le même protocole que celui décrit dans l'exemple 3.2 a été utilisé afin d'effectuer un marquage de la protéine p63 à l'aide d'un anticorps primaire anti-p63, mouse monoclonal TEBU Santa Cruz. Le marquage par anticorps primaire est suivi par un marquage secondaire par un anticorps marqué à la fluorescence. Le montage de la lame dans un milieu adéquate puis la lecture par un microscope à épifluorescence permettront d'apprécier l'intensité du marquage. Ce marquage est réalisé en parallèle sur des biopsies traitées ou non avec l'extrait actif afin d'apprécier l'effet de ce dernier sur lesdites biopsies.

### Résultats :

Les follicules traités pendant 48 heures avec l'extrait peptidique présentent un marquage de la protéine p63 très augmenté par rapport aux follicules non traités. En effet, grâce à une quantification de la fluorescence par un logiciel adapté, on constate une hausse de 13,6 % du marquage p63 dans les follicules traités. Or, il est connu que la protéine p63 est un marqueur des cellules souches folliculaires relié à la capacité de ces cellules à générer un follicule pileux.

### 3.5 Conclusions

Les 4 marquages effectués sur les biopsies de cheveux nous ont permis de démontrer que le traitement des follicules pileux par l'extrait peptidique de riz non fermenté permettait :
- d'augmenter la quantité de protéines qui sont des marqueurs des cellules souches folliculaires ;
- par conséquent, de favoriser le micro-environnement des cellules souches et ainsi leur protection ;
- et finalement de favoriser la différenciation et donc l'entrée en phase anagène des cellules souches folliculaires afin de stimuler au mieux la pousse des cheveux et/ou ralentir leur chute.

### Exemple 4 : Action de l'extrait peptidique sur les follicules en présence d'UV

Des biopsies de peaux (issues de liftings) présentant des cheveux sont cultivées de la même manière que des explants de peaux. Des biopsies de 6 mm sont réalisées au moyen de punch de biopsies et mises en culture sur des inserts dans un milieu WILLIAM E. en présence d'antibiotiques (Pénicilline 100 UI/ml-10 µg, streptomycine, 10 µg/ml d'insuline 10 nG/ml, d'Hydrocortisone et 2 mmol/L de L-Glutamine).

Les explants de peaux sont déposés dans des plaques 6 puits et les follicules sont traités en mettant 20 µl d'extrait à 1 % en contact sur les biopsies pendant 24 heures. Les biopsies sont ensuite soumises à une irradiation UVA à 5 J/cm², suivie d'une irradiation UVB à 200 mj/cm², et enfin remises pendant 24 heures supplémentaires en présence de l'extrait actif. Des boîtes contrôle avec des explants non traités par l'extrait peptidique actif mais irradiés servent de témoin. Une évaluation de l'état des cellules après irradiation UV est réalisée par un marquage hématoxyline/éosine.

### Résultats

La forme générale de l'outer root sheet (ORS) est irrégulière dans les follicules qui n'ont pas été prétraités et traités par l'extrait peptidique, alors que celle-ci est tout à fait régulière dans les follicules traités. En outre, les cellules des follicules pileux non traités présentent des dommages dus aux rayonnements UVA et UVB, dommages tels que l'apparition de vacuoles, d'oedèmes et de cellules apoptotiques. Toutes ces manifestations ne sont pas observées dans les follicules prétraités et traités par l'extrait peptidique. On en déduit donc un rôle protecteur de cet extrait peptidique de riz non fermenté sur les follicules pileux et, entre autre, sur les cellules souches de ces follicules.

### Exemple 5 : Action de l'hydrolysat peptidique sur la croissance des follicules

### Mesures de l'élongation des follicules

Des biopsies de peaux issues de liftings sont rasées, puis mises en culture dans un milieu approprié (milieu Wiliam E.) en présence ou non de l'hydrolysat peptidique actif à une concentration de 1 %. En absence de principe actif, les follicules sont mis en culture dans du PBS. Des photos sont réalisées à l'aide de la Vivacam (petite camera qui équipe le Vivascope) au temps zéro. Les biopsies sont remises en culture et des mesures d'élongation des follicules pileux sont réalisées aux jours 8, 14 et 21.

Les photos aux différents temps sont ensuite retraitées par le logiciel « Image Pro Analyzer® » qui nous permet de mesurer la taille (en µm) de chaque follicule séparément. L'élongation globale des follicules traités et non traités est mesurée et une étude statistique est réalisée et rassemblée dans le graph de la figure 1.

### Résultats :

A l'issue de ce test, on constate que l'application de l'hydrolysat peptidique de riz non fermenté possède une action sur la pousse des cheveux. En effet, il a permis une croissance du follicule pileux de manière plus importante par rapport à la croissance observée dans les follicules non traités.

### Exemple 6 : Mise en évidence de l'action protectrice d'un mélange d'un extrait peptidique de riz non fermenté avec un extrait peptidique de soja sur des cellules de biopsies de peau soumises à des rayonnements UVB

Le but de cette expérience est de mesurer les capacités de réparation d'un mélange comprenant un extrait peptidique de riz non fermenté en association avec un extrait peptidique de soja (ci-après dénommé extrait riz/soja) après irradiation par rayonnements UVB de biopsies de peau humaine. Il a été démontré auparavant que les résultats qui seront obtenus sur les cellules de biopsies de peaux sont corrélables à ceux qui seraient obtenus au niveau des cellules de l'ORS. Le rayonnement UV, et particulièrement UVB, entraîne des réactions de dimérisation qui ont lieu aux sites comprenant deux pyrimidines adjacentes (thymidine, cytosine). Plusieurs types de photoproduits sont alors formés, dont les dimères de cyclobutane-pyrimidine ou DCPs. Or, il est connu que lorsqu'une cellule est exposée à un stress génotoxique, son cycle de division est temporairement arrêté pour permettre la réparation de l'ADN et éviter ainsi l'apparition de mutations dans les générations suivantes de cellules. La prolifération cellulaire ne reprend qu'ensuite. Si la fréquence ou la quantité des dommages est trop élevée, ou encore la réparation inefficace, les cellules enclenchent un processus de mort programmée, l'apoptose. Ce type de phénomène est observé au niveau des cellules des follicules pileux et conduit à la mort du cheveu ou à sa perte d'épaisseur. Par conséquent, en mesurant par immunomarquage, à l'aide d'un anticorps anti-DCPs, la quantité de photoproduits formés, il est possible d'évaluer l'efficacité d'un composé ayant une action réparatrice sur l'ADN des cellules.

### Protocole d'immunomarquage des DCPs sur des biopsies de peau soumises à des UVB

Des biopsies de peau humaine sont mises en culture à l'interface air/liquide et prétraitées avec l'extrait riz/soja à 1 % pendant 24 heures, ou avec le placebo (PBS) pour la condition contrôle. Ces biopsies sont soumises à des irradiations par rayonnements UVB à raison de 200 mJ/cm². Après irradiation, les biopsies sont remises en culture pendant 24 heures avec seulement application de PBS (c'est-à-dire sans actif).

Pour le marquage des dimères cyclobutane-pyrimidine, les biopsies de peau sont incluses dans de la paraffine, et des coupes histologiques de 3 µm d'épaisseur sont effectuées. Les lames sont déparaffinées, hydratées puis soumises à un immunomarquage par un anticorps dirigé contre les dimères cyclobutane-pyrimidine (MBL D194-1, mouse monoclonal) puis par un anticorps secondaire adapté (Invitrogen A21202) couplé à un marqueur fluorescent. Les coupes de peau sont alors examinées au microscope à Epi-fluorescence (Nikon Eclipse E 80i microscope).

En condition contrôle, la fluorescence observée est plus intense lorsque les biopsies ont été soumises aux rayonnements UVB. Dans la condition testée avec prétraitement par l'extrait de riz/soja, on observe une fluorescence beaucoup moins intense que dans la condition contrôle avec UVB.

### Conclusions :

L'extrait de riz/soja a permis une meilleure protection des biopsies de peau car moins de dommages de type DCPs sont observés dans les biopsies prétraitées par ledit extrait. On peut donc extrapoler ces résultats et conclure que l'extrait de riz/soja selon l'invention aurait les mêmes effets sur les cellules de l'ORS et protègerait celles-ci des rayonnements UV.

### Exemple 7 : Préparation de compositions

### 1. Sérum pour la pousse des cheveux

Disperser le Natrosol 250HHR et le Disodium EDTA dans l'eau sous agitation. Chauffer à 50-60 °C, et agiter jusqu'à obtenir un aspect uniforme. Ajouter le Styleze^{®} CC-10 et agiter jusqu'à obtenir un aspect uniforme. Laisser refroidir à température ambiante et ajouter les ingrédients dans l'ordre de la liste en agitant jusqu'à obtenir un aspect uniforme entre chacun d'eux.

| **Formulations** | | **N° 1** | **N° 2** | |
|---|---|---|---|---|
| **Nom INCI** | **Nom commercial** | **% massique** | **% massique** | **Fournisseur** |
| Eau | | Q.S. | Q.S. | |
| Hydroxyethylcellulose | Natrosol 250HHR | 0,35 | 0,50 | Hercules/ Aqualon |
| Disodium EDTA | Dissolvine NA-2S | 0,05 | 0,05 | Akzo Nobel |
| VP/DMAPA Acrylates Copolymères | Styleze® CC-10 | 5,00 | 5,00 | ISP |
| Quaternium-26 | Ceraphyl® 65 | 1,00 | 1,00 | ISP |
| Panthénol | Ritapan DL | 0,15 | 0,15 | RITA |
| Propylène Glycol Urée Diazolidinyle Iodopropynyl Butylcarbamate | Liquid Germall® Plus | 0,50 | 0,50 | ISP |
| | Hydrolysat selon l'exemple 2 | 1,00 | 1,00 | ISP |
| Total | | 100,00 | 100,00 | |

Appliquer le produit sur le cuir chevelu humide. Masser pour répartir uniformément le produit. Le sérum favorise la pousse et/ou la repousse des cheveux tout en les rendant plus nerveux.

### 2. Lait traitant antichute de cheveux

Mettre l'eau dans un récipient convenable et commencer l'agitation. Ajouter le Gafquat 755N et le Liquid Germall Plus et agiter jusqu'à obtenir un aspect uniforme. Ajouter le RapiThix A-60 et agiter jusqu'à obtenir un aspect uniforme (environ 15 minutes). Ajouter l'hydrolysat selon l'exemple 1 et agiter jusqu'à obtenir un aspect uniforme. Disposer le produit dans un vaporisateur non-aérosol équipé d'une pompe Calmar pump Mark VI WL31.

| **Formulations** | | **N° 1** | **N° 2** | |
|---|---|---|---|---|
| **Nom INCI** | **Nom commercial** | % **massique** | % **massique** | **Fournisseur** |
| Eau dé-ionisée | - | Q.S. | Q.S. | |
| Polyquaternium-11 | Gafquat® 755N | 1,25 | 2,00 | ISP |
| Propylène Glycol Urée Diazolidinyl Iodopropynyl Butylcarbamate/ | Liquid Germall® Plus | 0,50 | 0,50 | ISP |
| Sodium Polyacrylate Polydecene Hydrogéné Trideceth-6/ | RapiThix™ A-60 | 0,50 | 0,50 | ISP |
| | Hydrolysat selon l'exemple 1 | 0,1 | 0,5 | ISP |
| | Total | 100,00 | 100,00 | |

Le produit est conçu pour être vaporisé sur le cuir chevelu et sur cheveux humides. Masser pour répartir uniformément le produit. Le lait ainsi proposé permet de lutter contre la chute des cheveux tout en les rendant lisses et faciles à coiffer.

## Revendications

1. Utilisation cosmétique d'une composition comprenant au moins un hydrolysat peptidique de riz non fermenté provenant de l'hydrolyse des protéines des grains de riz débarrassés de leur enveloppe par une étape de décorticage, qui comprend au moins 70 % de peptides de taille inférieure à 6 kDa, en tant qu'agent actif pour freiner, limiter la chute des cheveux et/ou stimuler leur croissance en augmentant la quantité de protéines marqueurs des cellules souches adultes folliculaires et en favorisant la différenciation de ces cellules.

2. Utilisation selon la revendication 1, pour activer la pousse des cils.

3. Utilisation selon l'une quelconque des revendications précédentes, **caractérisée en ce que** les grains proviennent de l'espèce de riz *Oryza Sativa L.*

4. Utilisation selon l'une quelconque des revendications précédentes, **caractérisée en ce que** l'hydrolysat est solubilisé dans un ou plusieurs solvants physiologiquement acceptables choisi parmi l'eau, le glycérol, l'éthanol, le propylène glycol, le butylène glycol, le dipropylène glycol, les diglycols éthoxylés ou propoxylés, les polyols cycliques ou tout mélange de ces solvants.

5. Utilisation selon l'une quelconque des revendications précédentes, **caractérisée en ce que** l'hydrolysat solubilisé comprend au moins entre 1,5 et 4 g/l de peptides, et préférentiellement au moins entre 2 et 3 g/l de peptides.

6. Utilisation selon l'une quelconque des revendications précédentes, caractérisée en ce l'hydrolysat comprend au moins 85% de peptides de taille inférieure à 6 kDa.

7. Utilisation selon l'une quelconque des revendications précédentes, **caractérisée en ce que** l'hydrolysat solubilisé est utilisé en une quantité représentant de 0,001 % à 5 % du poids total de la composition, et préférentiellement en une quantité représentant de 0,01 % à 1 % du poids total de la composition.

8. Utilisation selon l'une quelconque des revendications précédentes, **caractérisée en ce que** la composition se présente sous une forme adaptée à l'application par voie topique.

9. Utilisation selon l'une quelconque des revendications précédentes, **caractérisée en ce que** la composition comprend en outre un composé choisi parmi des vitamines, d'autres hydrolysats peptidiques végétaux, le minoxidil, des esters de l'acide nicotinique, des oligo-éléments, des agents anti-inflammatoires, de l'acide rétinoïque ou ses dérivés, du rétinol, des inhibiteurs de la 5α-réductase ou des composés peptidiques issus de la synthèse chimique.

10. Utilisation selon la revendication 9, **caractérisée en ce que** la composition comprend un hydrolysat peptidique de soja.

11. Utilisation selon la revendication 10, **caractérisée en ce que** l'extrait peptidique de riz non fermenté et l'extrait peptidique de soja sont présents à des concentrations comprises entre 10 et 90 %.

## Patentansprüche

1. Kosmetische Verwendung einer Zusammensetzung, die mindestens ein Peptidhydrolysat von nicht fermentiertem Reis umfasst, das aus der Hydrolyse von Proteinen von Reiskörnern, deren Schale durch einen Schälschritt entfernt wurde, stammt und das mindestens 70 % Peptide mit einer Größe von weniger als 6 kDa umfasst, als Wirkstoff zum Verlangsamen und Einschränken von Haarausfall und/oder Stimulieren von Haarwachstum durch Erhöhen der Menge an Markerproteinen von adulten Follikelstammzellen und durch Begünstigen der Differenzierung dieser Zellen.

2. Verwendung nach Anspruch 1 zum Aktivieren von Wimpernwachstum.

3. Verwendung nach einem der vorhergehenden Ansprüche, **dadurch gekennzeichnet, dass** die Körner von der Reisspezies *Oryza sativa L.* stammen.

4. Verwendung nach einem der vorhergehenden Ansprüche, **dadurch gekennzeichnet, dass** das Hydrolysat in einem oder mehreren physiologisch akzeptablen Lösungsmitteln solubilisiert wird, die aus Wasser, Glycerin, Ethanol, Propylenglykol, Butylenglykol, Dipropylenglykol, ethoxylierten oder propoxylierten Diglykolen, cyclischen Polyolen oder einem beliebigen Gemisch dieser Lösungsmittel ausgewählt sind.

5. Verwendung nach einem der vorhergehenden Ansprüche, **dadurch gekennzeichnet, dass** das solubilisierte Hydrolysat mindestens zwischen 1,5 und 4 g/l Peptide und vorzugsweise mindestens zwischen 2 und 3 g/l Peptide umfasst.

6. Verwendung nach einem der vorhergehenden Ansprüche, **dadurch gekennzeichnet, dass** das Hydrolysat mindestens 85 % Peptide mit einer Größe von weniger als 6 kDa umfasst.

7. Verwendung nach einem der vorhergehenden Ansprüche, **dadurch gekennzeichnet, dass** das solubilisierte Hydrolysat in einer Menge verwendet wird, die 0,001 % bis 5 % des Gesamtgewichts der Zusammensetzung ausmacht, und vorzugsweise in einer Menge verwendet wird, die 0,01 % bis 1 % des Gesamtgewichts der Zusammensetzung ausmacht.

8. Verwendung nach einem der vorhergehenden Ansprüche, **dadurch gekennzeichnet, dass** die Zusammensetzung in einer Form vorliegt, die zur topischen Anwendung geeignet ist.

9. Verwendung nach einem der vorhergehenden Ansprüche, **dadurch gekennzeichnet, dass** die Zusammensetzung außerdem eine Verbindung umfasst, die aus Vitaminen, anderen pflanzlichen Peptidhydrolysaten, Minoxidil, Nikotinsäureestern, Oligoelementen, entzündungshemmenden Mitteln, Retinsäure oder deren Derivaten, Retinol, 5α-Reduktasehemmern oder Peptidverbindungen, die aus einer chemischen Synthese hervorgehen, ausgewählt ist.

10. Verwendung nach Anspruch 9, **dadurch gekennzeichnet, dass** die Zusammensetzung ein Soja-Peptidhydrolysat umfasst.

11. Verwendung nach Anspruch 10, **dadurch gekennzeichnet, dass** der Peptidextrakt von nicht fermentiertem Reis und der Soja-Peptidextrakt in Konzentrationen vorliegen, die zwischen 10 und 90 % liegen.

## Claims

1. Cosmetic use of a composition comprising at least one non-fermented rice peptide hydrolysate from the hydrolysis of rice grain proteins that are husked by a shelling step, which comprises at least 70% of peptides with a size of less than 6 kDa, as an active agent in order to slow down and limit hair loss and/or stimulate hair growth by increasing the quantity of marker proteins of follicular adult stem cells and by favouring differentiation of these cells.

2. Use according to claim 1, in order to activate the growth of eyelashes.

3. Use as claimed in any preceding claim, **characterised in that** the grains come from the rice species *Oryza Saliva L.*

4. Use as claimed in any preceding claim, **characterised in that** the hydrolysate is solubilised in one or several physiological acceptable solvents chosen from water, glycerol, ethanol, propylene glycol, butylene glycol, dipropylene glycol, ethoxylated or propoxylated diglycols, cyclic polyols, or any mixture of these solvents.

5. Use as claimed in any preceding claim, **characterised in that** the solubilised hydrolysate comprises at least between 1.5 and 4 g/l of peptides, and preferentially at least between 2 and 3 g/l of peptides.

6. Use as claimed in any preceding claim, **characterised in that** the hydrolysate comprises at least 85% of peptides of size less than 6 kDa.

7. Use as claimed in any preceding claim, **characterised in that** the solubilised hydrolysate is used in a quantity that represents from 0.001% to 5% of the total weight of the composition, and preferentially in a quantity that represents from 0.01% to 1% of the total weight of the composition.

8. Use as claimed in any preceding claim, **characterised in that** the composition has the form adapted to topical application.

9. Use as claimed in any preceding claim, **characterised in that** the composition further comprises a compound chosen from vitamins, other plant peptide hydrolysates, minoxidil, esters of nicotinic acid, trace elements, anti-inflammatory agents, retinoic acid or its derivatives, retinol, inhibitors of 5α-reductase or peptide compounds derived from chemical synthesis.

10. Use according to claim 9, **characterised in that** the composition comprises a soy peptide hydrolysate.

11. Use according to claim 10, **characterised in that** the non-fermented rice peptide hydrolysate and the soy peptide extract are present at concentrations between 10 and 90%.
